# EUROPEAN PATENT APPLICATION

(11) **EP 4 550 955 A1**
(43) Date of publication of application: **07.05.2025**
(21) Application number: 23871414.1
(22) Date of filing: 25.07.2023
(51) Int. Cl.: H05K 7/00, C12M 1/00, H02G 3/22

(54) **CABLE COVER AND CONSTANT-TEMPERATURE UNIT PROVIDED WITH SAME**

(30) Priority: 29.09.2022 JP 2022156335
(71) Applicant: PHC Holdings Corporation, Tokyo 100-8403 (JP)
(72) Inventor: NAKAMAE, Kenta, (JP); HIRAI, Hiroki, (JP); HORIMOTO, Nobuo, (JP); HORIKAWA, Kiyohiro, (JP); HIRATSUKA, Takashige, (JP); WATANABE, Hidenori, (JP); IKETANI, Seiitirou, (JP)
(74) Representative: Grünecker Patent- und Rechtsanwälte PartG mbB
(86) International application number: PCT/JP2023/027152
(87) International publication number: WO 2024/070171

(57) **Abstract**

A cable cover (10) fixes, in a measurement hole (20) provided in the wall surface of an incubator (30), a cable (C) that comes out of an internal space (S2), through a measurement hole (20), and into an external space (S1). The cable cover (10) includes a grommet (11), a core sleeve (12), and a heater (13). The grommet (11) holds the cable (C). The substantially cylindrical core sleeve (12) is disposed around the outer periphery of the grommet (11). The heater (13) is disposed on the core sleeve (12) and applies heat to the cable (C) held in the grommet (11) via the core sleeve (12).

## Description

### TECHNICAL FIELD

The present invention relates to a cable cover that fixes a cable that goes from the inside to the outside through an opening provided in the wall of a constant-temperature unit, to the wall surface of the constant-temperature unit, as well as a constant-temperature unit equipped with this cable cover, for example.

### BACKGROUND ART

Culture devices have been widely used in recent years for culturing cells, microorganisms, and so forth. These culture devices are provided with a heater or the like to create a high-temperature and high-humidity environment in the culture chamber.

For instance, Patent Literature 1 discloses a cover that is attached to a wall when a cable or the like is being passed through a constant-temperature unit such as a working device or a storage device, as well as a constant-temperature unit in which this cover is used.

This cover is used in a constant-temperature unit having an internal chamber that is surrounded by a plurality of walls, at least one of which has a hole in it, and a temperature and humidity control unit that controls the temperature and humidity in the internal chamber, and this cover is attached so as to cover up the hole in the wall, with a space between the wall and the cover, and has one or more hole portions that are used to pass a string-like object extending from the outside of the internal chamber to the inside through the hole, and further has a surrounding portion that makes sealing contact with the wall, a contact portion that makes sealing contact with the string-like object in the hole, and a seal body that makes sealing contact with the hole, and the string-like object is passed through the seal body while preserving the sealed state, and part of the string-like object is held within the space.

### CITATION LIST

### PATENT LITERATURE

Patent Literature 1: JP-A 2009-178070 (Japanese Patent No. 5,017,133)

### SUMMARY

### (TECHNICAL PROBLEM)

However, the following problem is encountered with the above-mentioned conventional cover.

With the cover disclosed in the above publication, a heater is built into the inner wall of the constant-temperature unit, and the temperature inside the storage chamber can be kept constant by the heater and thermal insulating material. The interior of the cable storage section and a part of the cable stored inside the cable storage section are heated by thermal conduction from the heater, which prevents condensation on the cover.

With this configuration, however, since a heater for adjusting the temperature inside the constant-temperature unit is installed on the inner wall of the device to heat part of the cable inside the cover and prevent condensation, there is the risk that the heat from the heater may not be transmitted directly to the cable.

It is an object of the present invention to provide a cable cover that fixes a cable going from the inside to the outside through an opening provided in the wall of a constant-temperature unit, wherein condensation can be prevented more effectively from occurring near an opening, as well as a constant-temperature unit equipped with this cable cover.

### (SOLUTION TO PROBLEM)

The cable cover according to the first invention is a cable cover that fixes, in an installation hole provided in a wall surface of a constant-temperature unit, a cable coming out of the internal space, through the installation hole, and into the external space, the cable cover including a holding member, a first sleeve member, and a heating unit. The holding member holds the cable. The substantially cylindrical first sleeve member is disposed around the outer periphery of the holding member. The heating unit is disposed on the first sleeve member and applies heat to the cable held by the holding member via the first sleeve member.

Here, heat is applied from the heating unit disposed on the first sleeve member to the cable that goes from the internal space to the external space through the installation hole provided in the wall of the constant-temperature unit and is held by the holding member, thereby heating part of the cable that is fixed in the installation hole provided in the wall of the constant-temperature unit.

Here, the constant-temperature unit is, for example, a box-shaped device such as an incubator that creates a high-temperature and high-humidity environment in the internal space of the housing and controls the internal space to maintain a constant environment.

The first sleeve member is, for example, a component made of a metal having high thermal conductivity, such as aluminum, and is provided with a heating unit such as a heater on its outer peripheral surface.

The heating unit is, for example, a sheet-shaped heater, and is switched between heating and non-heating states by turning the supply of power on or off.

This effectively prevents condensation from occurring near the installation hole through which the cable is passed into the constant-temperature unit, even though the inside of the constant-temperature unit is an environment of high temperature and humidity and the temperature is low in the external space, for example.

The cable cover according to the second aspect of the present invention is a cable cover that fixes, in an installation hole provided in a wall surface of a constant-temperature unit, a cable coming out of the internal space, through the installation hole, and into the external space, the cable cover including a first holding member, a second holding member, and a heating unit. The first holding member holds the cable. The second holding member is disposed so as to come into contact with the first holding member each other in the extension direction of the cable. The heating unit is disposed on the first holding member and applies heat to the cable held by the first holding member via the first holding member.

Here, heat is applied from a heating unit disposed on the first holding member to a cable that goes from the internal space to the external space through an installation hole provided in the wall of the constant-temperature unit, and is held by the holding member, thereby heating part of the cable that is fixed in the installation hole provided in the wall of the constant-temperature unit.

Here, the constant-temperature unit is, for example, a box-shaped device such as an incubator that produces an environment of high temperature and humidity in the internal space of the housing and controls the internal space to maintain a constant environment.

The first holding member is, for example, a component made of a metal having high thermal conductivity, such as aluminum, and is provided with a heating unit such as a heater.

The heating unit is, for example, a cylindrical cartridge-type heater, and switching between heating and non-heating states is accomplished by turning the power supply on or off.

This makes effectively prevents condensation from occurring near the installation hole through which the cable is pulled into the constant-temperature unit, even though the inside of the constant-temperature unit is an environment of high temperature and humidity, and the temperature in the external space is low, for example.

The cable cover according to the third invention is a cable cover that fixes, in an installation hole provided in a wall surface of a constant-temperature unit, a cable coming out of the internal space, through the installation hole, and into the external space, wherein a holding member holds the cable. A substantially cylindrical sleeve member is disposed around the outer periphery of the holding member and has a first threaded portion formed on the outer peripheral surface of the sleeve member along the lengthwise direction of the cable. A fixing member has a substantially annular main body part and a second threaded portion formed on the inner peripheral side of the main body part and meshing with the first threaded portion, and is fixed at a position corresponding to the length of the installation hole in a state in which the second threaded portion is meshed with the first threaded portion.

Here, when the cable that has been passed from the internal space to the external space through the installation hole provided in the wall of the constant-temperature unit and is held by the holding member is attached in the installation hole provided in the wall of the constant-temperature unit, the cable is fixed using a fixing member that meshes with a first threaded portion formed on the outer peripheral surface of the sleeve member along the lengthwise direction of the cable. At this point, the fixing member is fastened at a position on the first threaded portion according to the length of the installation hole.

This allows the cable to be attached to installation holes of different lengths by fastening the fixing member, which meshes with the first threaded portion formed along the lengthwise direction of the cable, at a position corresponding to the length of the installation hole.

The cable cover according to the fourth invention is the cable cover according to the first invention, further equipped with a substantially cylindrical second sleeve member that is attached so as to cover the outer periphery of the first sleeve member and the heating unit.

This allows the second sleeve member to be attached so as to cover the outer periphery of the first sleeve member where the heating unit is disposed.

The cable cover according to the fifth invention is the cable cover according to the fourth invention, wherein the second sleeve member has a first threaded portion that is formed on the substantially cylindrical outer peripheral surface along the lengthwise direction of the cable. The cable cover is further equipped with a fixing member that has a substantially annular main body part and a second threaded portion formed on the inner peripheral side of the main body part and meshing with the first threaded portion, and that is fixed at a position corresponding to the length of the installation hole in a state in which the second threaded portion is meshed with the first threaded portion.

This allows a cable to be attached to installation holes of different lengths by fastening the fixing member, which meshes with the first threaded portion provided on the outer periphery of the second sleeve member, at a position corresponding to the length of the installation hole.

The cable cover according to the sixth invention is the cable cover according to the fourth invention, wherein the second sleeve member has a first end on the outdoor side of the constant-temperature unit, and a second end on the indoor side. The cable cover is further equipped with an intermediate member that is disposed on the side of the second end of the second sleeve member and has substantially the same outside diameter as the second sleeve member, and a cap member that is attached so as to cover the second end of the second sleeve member and the outer peripheral surface of the intermediate member.

Consequently, the side of the second end of the second sleeve member can be covered by attaching the cap member so as to cover the second end on the indoor side of the second sleeve member and the intermediate member disposed near the second end.

The cable cover according to the seventh invention is the cable cover according to the sixth invention, wherein mounting of the cap member causes the intermediate member to be deformed, forming a waterproofing mechanism in the installation hole.

Consequently, a waterproofing mechanism can be formed near the cable installed in the installation hole by deforming the intermediate member when the cap member is mounted.

The cable cover according to the eighth invention is The cable cover according to the sixth or seventh invention, further equipped with a sealing member that is mounted to the outer peripheral surface of the cap member, is elastic, and is inserted into the installation hole to seal off the internal space.

This ensures an airtight seal in the internal space of the constant-temperature unit, for example, and prevents problems such as hot, moist air leaking out from the internal space to the external space.

The cable cover according to the ninth invention is the cable cover according to the first or third invention, wherein the holding member has a plurality of holding portions that hold a plurality of the cables.

This allows a single cable cover to be attached to an installation hole in a state in which a plurality of cables are held.

The cable cover according to the tenth invention is the cable cover according to the ninth invention, further equipped with a blocking member that blocks off the end of a holding portion that is not holding the cable, among the plurality of holding portions of the holding member.

Consequently, when a holding member capable of holding multiple cables is used, if all of the holding portions are not being used, the ends of the holding portions can be blocked off with a blocking member to keep the internal space of the constant-temperature unit in a sealed state.

The cable cover according to the eleventh invention is the cable cover according to the first or third invention, wherein the holding member is elastic.

Consequently, the cable can be held while being appropriately deformed according to the shape of the cable by holding the cable with an elastic holding member. This prevents gaps from occurring near the cable and maintains the airtightness of the internal space of the constant-temperature unit.

The cable cover according to the twelfth invention is The cable cover according to the eleventh invention, wherein the holding member has a press-fit portion that makes contact with the inner peripheral surface of the installation hole when installed in the installation hole, and is held by elastic deformation.

This allows the cable cover to be easily fixed to the installation hole by utilizing the elasticity of the holding member to press-fit the holding member so that its outer peripheral surface makes contact with the inner peripheral surface of the installation hole.

The cable cover according to the thirteenth invention is The cable cover according to the eleventh invention, wherein the holding member has a holding portion that holds the cable, and a cut portion that is provided in the outer peripheral surface thereof and that elastically deforms when the cable is held by the holding portion, thereby moving the cable to the holding portion.

Consequently, the cable can be stably held inside the holding member by elastically deforming the cut portion provided on the outer peripheral surface of the holding member and inserting the cable into the holding portion.

The cable cover according to the fourteenth invention is The cable cover according to the second invention, wherein the first holding member has a substantially cylindrical insertion hole provided in the center thereof, and the heating unit is inserted into the insertion hole.

Consequently, heat coming off the heating unit can be transferred to the cable held by the first holding member by inserting the heating unit into the insertion hole provided in the center of the first holding member.

The cable cover according to the fifteenth invention is the cable cover according to the fourteenth invention, wherein the first holding member has a holding portion provided on the outer peripheral side of the insertion hole, and the cable is held in this holding portion.

This allows the first holding member to transfer heat from the heating unit inserted into the insertion hole provided in the center to the cable held in the holding portion provided on the outer peripheral side of the insertion hole.

The cable cover according to the sixteenth invention is the cable cover according to the fifteenth invention, wherein the first holding member has a plurality of the holding portions, and the plurality of holding portions are recesses into which the cables are fitted from the outer peripheral side of the first holding member.

This allows a plurality of cables to be held in a plurality of recesses (holding portions) provided on the outer peripheral side of the first holding member.

The cable cover according to the seventeenth invention is the cable cover according to the second invention, wherein the second holding member is formed by a substantially cylindrical elastic member.

Consequently, a cable can be held by the elastic, substantially cylindrical second holding member.

The cable cover according to the eighteenth invention is the cable cover according to the seventeenth invention, wherein the second holding member has a holding portion that holds the cable, and a cut portion that is provided on the outer peripheral surface of the holding portion and elastically deforms when the cable is held by this holding portion, thereby moving the cable to the holding portion.

Consequently, the cable can be stably held inside the second holding member by elastically deforming the cut portioned portion provided on the outer peripheral surface of the second holding member and inserting the cable into the holding portion.

The cable cover according to the nineteenth invention is the cable cover according to the seventeenth invention, wherein the second holding member has a press-fit portion that makes contact with the inner peripheral surface of the installation hole when installed in the installation hole and is held by elastic deformation.

Consequently, the cable cover can be easily fixed to the installation hole by utilizing the elasticity of the second holding member to press-fit the second holding member so that its outer peripheral surface makes contact with the inner peripheral surface of the installation hole.

The cable cover according to the twentieth invention is the cable cover according to the second invention, wherein the first holding member is formed from a metal material having high thermal conductivity.

Consequently, since the first holding member is made of a metallic material such as aluminum, for example, the heat coming off the heating unit can be efficiently transferred to the cable.

The constant-temperature unit according to the twenty-first invention is equipped with the cable cover according to any of the first to third inventions, an attachment fixture that is provided to an installation hole to which the cable cover is attached, and a housing that includes the wall surface provided with the installation hole to which the mounting fixture is attached.

Consequently, a constant-temperature unit can be provided with which condensation can be more effectively prevented from occurring near the opening in a cable cover that fixes a cable coming from the inside to the outside through an opening provided in the wall of the constant-temperature unit.

### (EFFECTS)

According to the cable cover of the present invention, in a cable cover that secures a cable pulled from the inside to the outside through an opening provided in the wall of a constant-temperature unit, the occurrence of condensation near the opening can be more effectively prevented.

### BRIEF DESCRIPTION OF THE DRAWINGS

Fig. 1 is an oblique view of the external configuration of an incubator to which a cable cover according to an embodiment of the present invention is attached;
Fig. 2A is a rear view of the constant-temperature unit in Fig. 1, and Fig. 2B is a cross-sectional view along the A-A line of the constant-temperature unit in Fig. 2A;
Fig. 3 is a cross-sectional view of a cable cover that is fixed to a measurement hole attached to the constant-temperature unit of Fig. 1 and holds a cable;
Fig. 4A is a front view of the cable cover holding the cable of Fig. 3, and Fig. 4B is a cross-sectional view along the B-B line in Fig. 4A;
Fig. 5 is an overall oblique view of the configuration of the cable cover holding the cable in Fig. 3;
Fig. 6 is an exploded oblique view of the configuration of components included in the cable cover of Fig. 5;
Fig. 7 is an assembly diagram showing a state in which the grommet included in the component configuration in Fig. 6 is attached to a cable;
Fig. 8 is an assembly diagram showing a state in which a sleeve member is mounted around the outer periphery of the grommet in Fig. 7;
Fig. 9 is an assembly diagram showing a state in which a nut member is attached around the outer periphery of the sleeve member in Fig. 8;
Fig. 10 is an assembly diagram showing a state in which a distal end cap is attached to the distal end of the sleeve member in Fig. 9;
Fig. 11 is an assembly diagram showing a state in which the cable cover holding the cable in Fig. 10 has been inserted into a measurement hole;
Fig. 12 is an assembly diagram showing a state in which a sealing member has been mounted from the internal space side of an incubator in the sleeve member of the cable cover inserted into the measurement hole in Fig. 11;
Fig. 13 is an assembly diagram showing a state in which the sleeve member has been pulled from the external space side of the constant-temperature unit in the state shown in Fig. 12;
Fig. 14 is an assembly diagram showing a state in which the nut member has been moved from the state shown in Fig. 13 to a position according to the size of the measurement hole, and then tightened;
Figs. 15A and 15B are side views of cable covers mounted to measurement holes of different lengths;
Fig. 16A is a cross-sectional view showing a tapered portion provided on the inner peripheral surface side of the distal end cap, Fig. 16B is a cross-sectional view showing a state in which the distal end cap is mounted to the distal end of an outer sleeve, and Fig. 16C is a cross-sectional view showing elastic deformation of the intermediate member when the distal end cap is mounted to the distal end of the outer sleeve;
Fig. 17 is an oblique view of the configuration of the cable cover according to a second embodiment of the present invention;
Fig. 18 is an exploded oblique view of the component configuration of the cable cover shown in Fig. 17;
Fig. 19 is a cross-sectional view showing a state in which the cable cover in Fig. 17 has been fixed to the measurement hole of the constant-temperature unit of Fig. 1;
Fig. 20 is an oblique view of the configuration of the cable cover according to a third embodiment of the present invention;
Fig. 21 is an exploded oblique view of the component configuration of the cable cover in Fig. 20; and
Fig. 22 is a cross-sectional view showing a state in which the cable cover in Fig. 20 has been fixed to the measurement hole of the constant-temperature unit in Fig. 1 and holds a cable.

### DESCRIPTION OF THE EMBODIMENTS

### Embodiment 1

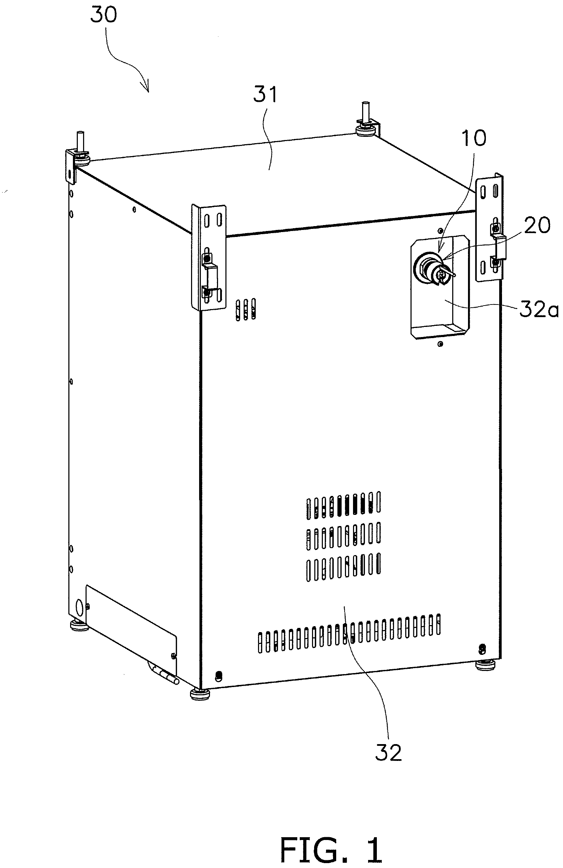

A cable cover 10 according to an embodiment of the present invention, and an incubator (constant-temperature unit) 30 including the same will now be described with reference to Figs. 1 to 16c.

In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims.

### (1) Cable Cover 10

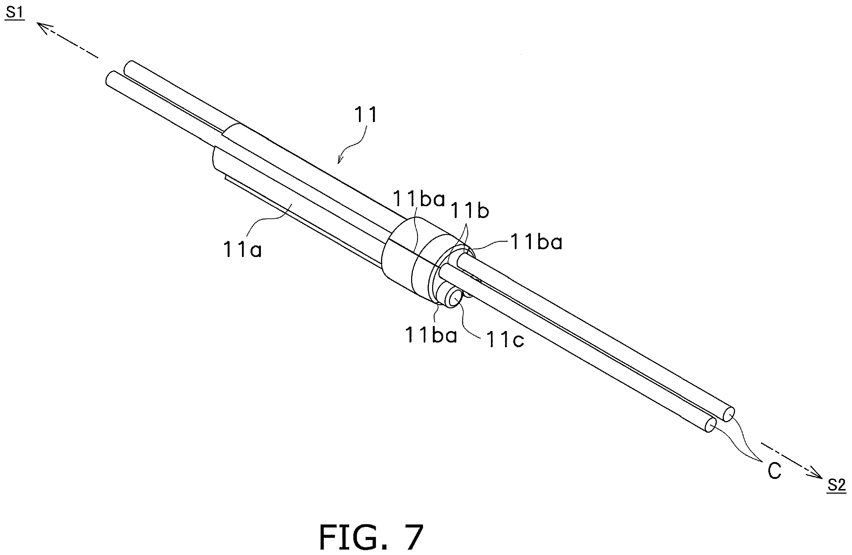

As shown in Fig. 1, the cable cover 10 in this embodiment fixes a cable C (see Fig. 7, etc.) that is passed from the internal space S2 to the external space S1 through a measurement hole (installation hole) 20 provided on the rear surface (wall surface) 32 of an incubator (constant-temperature unit) 30.

As shown in Fig. 1, in the constant-temperature unit 30, a measurement device (not shown) that makes various measurements of cell culture, etc., is placed in an internal space S2 formed in a housing 31, and the cable C for supplying power to the measurement device or transmitting and receiving various kinds of signal is passed through to the external space S1. The constant-temperature unit 30 is set such that the internal space S2 has an environment of high temperature and humidity environment (such as 37 degrees and 80%) in order to maintain the environment of the cells to be cultured. Accordingly, when the temperature in the external space S1 is low (such as 25 degrees or lower), condensation may occur near the measurement hole 20 on the cable C passed through from the internal space S2 to the external space S1, and on the inner wall near the measurement hole 20 in the internal space S2.

The anti-condensation structure provided by the cable cover 10 of this embodiment will be described in detail at a later point.

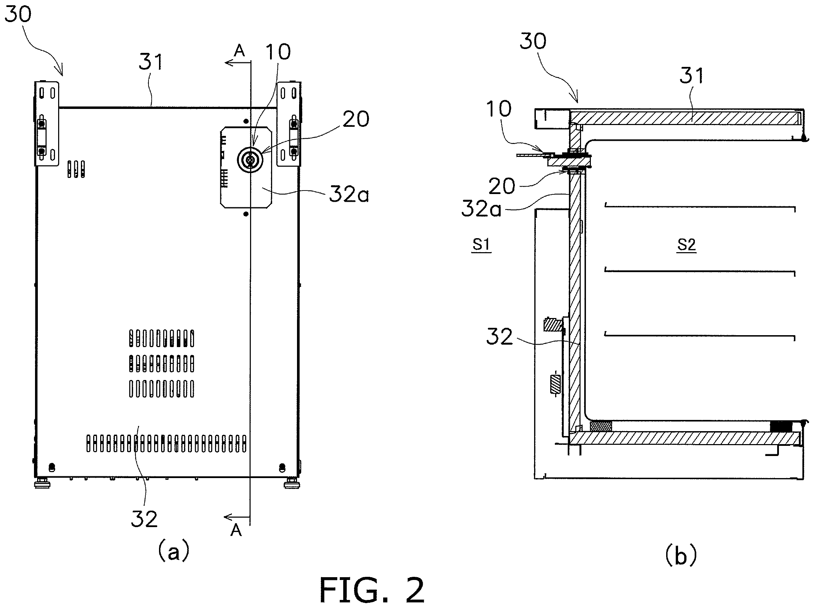

As shown in Figs. 2A and 2B, the constant-temperature unit 30 has a measurement hole 20 provided in a recess 32a provided on the rear surface 32 in order to pass the cable C from the internal space S2 of the housing 31 into the external space S1.

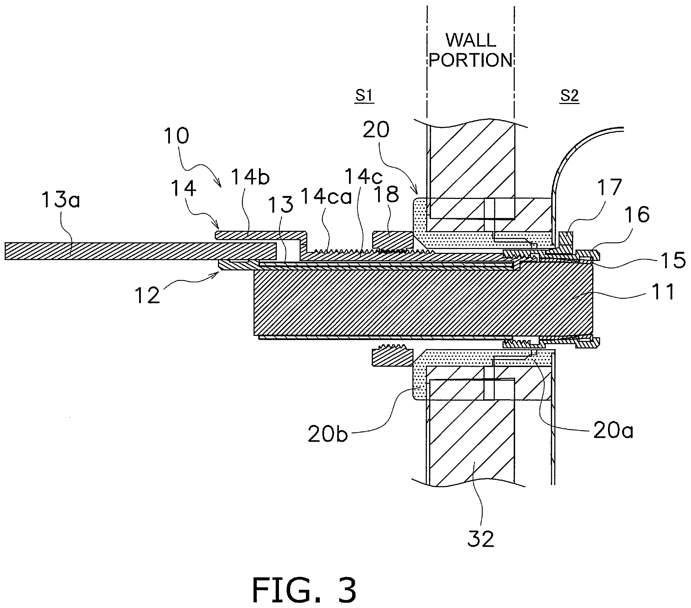

The measurement hole 20 is a cylindrical member to which the cable cover 10 holding the cable C is fixed, and is provided in a through-hole that goes through the rear surface 32. As shown in Fig. 3, the measurement hole 20 has a substantially cylindrical main body part 20a and a flange portion 20b.

As shown in Fig. 3, the substantially cylindrical main body part 20a is inserted from the external space S1 side along the inner peripheral surface of a through-hole formed in the rear surface 32, and then fixed.

As shown in Fig. 3, the flange portion 20b is formed on the external space S1 side of the substantially cylindrical main body part 20a, and is provided so as to protrude outward in the radial direction from the outer peripheral surface at the end of the main body part 20a on the external space S1 side. The flange portion 20b is fixed so as to make contact with the surface on the external space S1 side of the rear surface 32.

As shown in Fig. 3, the cable cover 10 holding the cable C (see Fig. 7, etc.) is attached to the measurement hole 20.

### Configuration of Cable Cover 10

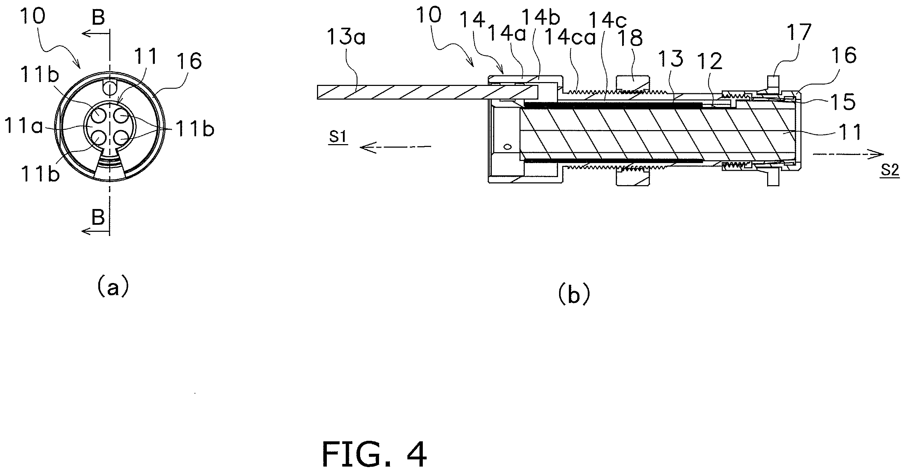

As shown in Figs. 4A and 4B, the cable cover 10 includes a grommet (holding member) 11, a core sleeve (first sleeve member, sleeve member) 12, a heater (heating unit) 13, an outer sleeve (second sleeve member, sleeve member) 14, an intermediate member 15, a distal end cap (cap member) 16, a sealing member 17, and a nut member (fixing member) 18.

The grommet (holding member) 11 is, for example, a member made of silicone rubber, and envelops the cable C while elastically deforming, and holds the outer peripheral portion of the cable C. The grommet 11 has a main body part 11a that constitutes the center portion of the cable cover 10, and a holding portion 11b that holds four cables C (see Fig. 7, etc.).

In this embodiment, a configuration is described in which the four cables C are held in the grommet 11, but the number of cables C held, that is, the number of holding portions 11b, is not limited to four.

The main body part 11a is a substantially cylindrical member and has grooves for holding the four cables C in its outer peripheral surface.

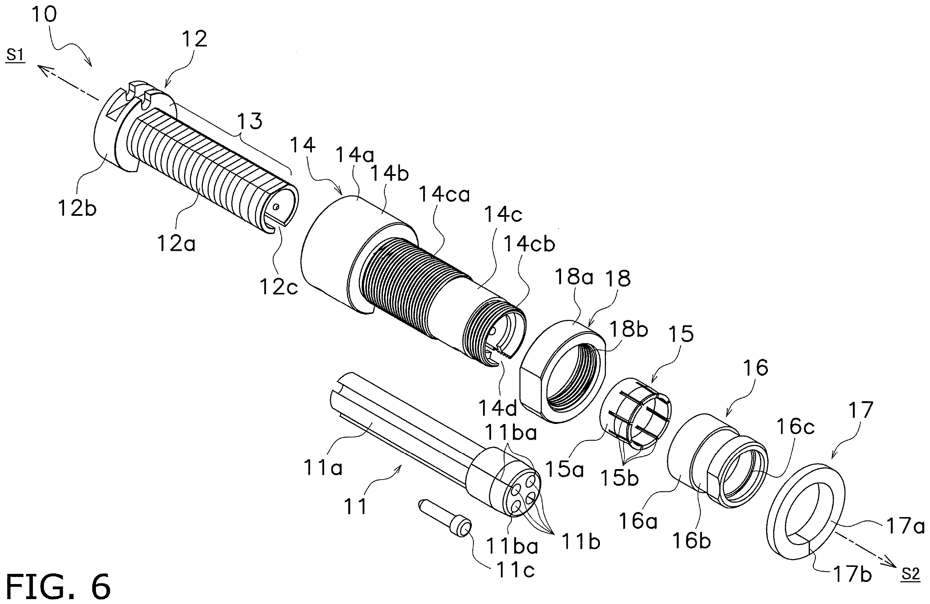

The holding portions 11b are provided at the end on the internal space S2 side of the main body part 11a, and hold the cables C that are passed through a cut portion 11ba (see Figs. 6 and 7) while being elastically deformed.

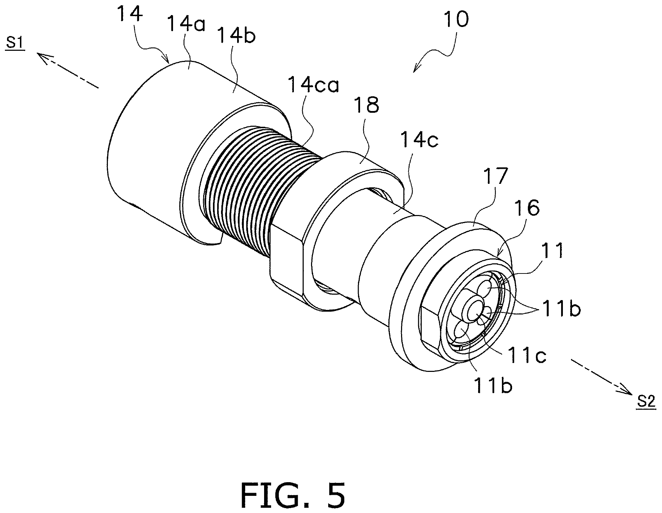

Of the holding portions 11b for holding the four cables C, those that are not holding a cable C allow the internal space S2 of the constant-temperature unit 30 to communicate with the external space S1, so in this embodiment, the holding portions 11b are blocked off with plugs (blocking members) 11c made of silicone rubber, as shown in Fig. 5.

The substantially cylindrical core sleeve (first sleeve member, sleeve member) 12 is, for example, a substantially cylindrical member made of aluminum, which has high thermal conductivity, and the grommet 11 is inserted and attached from the distal end thereof. A sheet-shaped heater 13 is wrapped around the outer peripheral surface of the core sleeve 12.

The main body part 12a is a substantially cylindrical member, and is provided with a flange portion 12b at the end on the external space S1 side.

As shown in Fig. 6, the flange portion 12b protrudes outward in the radial direction from the outer peripheral surface of the main body part 12a, and includes a portion that where a heater wire 13a (see Figs. 3 and 4b) of the heater 13 (discussed below) is passed through to the outside.

As shown in Fig. 6, a cut portion 12c is formed so as to split the outer peripheral surface of the substantially cylindrical main body part 12a in two along the axial direction.

This makes it easier to attach the cables C held by the grommet 11 when inserting the grommet 11 from the distal end of the core sleeve 12.

The heater (heating unit) 13 is disposed on the outer peripheral surface of the main body part 12a of the core sleeve 12, as shown in Fig. 6, in order to heat the portion of the cables C near the measurement hole 20, and applies heat to the cables C held in the grommet 11 enveloped by the core sleeve 12.

This heats the outer peripheral surface of the cables C near the measurement hole 20. Consequently, even if the temperature of the external space S1 is 25 degrees or lower, for example, it is possible to suppress the occurrence of condensation on the outer peripheral surface of the cables C near the measurement hole 20 and near the measurement hole 20 in the internal space S2 by heating the surface of the cables C to a temperature equal to or higher than the temperature of the internal space S2.

As shown in Fig. 3, etc., the heater wire 13a is connected to the heater 13 provided on the outer peripheral surface of the main body part 12a of the core sleeve 12 in order to supply power to the heater 13, and is passed through to the external space S1. If the temperature of the outer surface of a cable C to be heated exceeds a specific temperature setting, for example, the supply of power from the heater wire 13a to the heater 13 is halted, and once the temperature falls below the set temperature, the supply of power to the heater 13 is started.

The outer sleeve (second sleeve member, sleeve member) 14 is a substantially cylindrical member made of resin such as PC ( polycarbonate ), and is attached so as to cover the outer periphery of the core sleeve 12 and the heater 13. As shown in Fig. 6, the outer sleeve 14 has a main body part 14a, a large diameter portion 14b, a small diameter portion 14c, threaded portions 14ca and 14cb, and a cut portion 14d.

As shown in Fig. 6, the main body part 14a is configured by linking two substantially cylindrical members (the large diameter portion 14b and the small diameter portion 14c) having different outside diameters.

The large diameter portion 14b is a substantially cylindrical member on the side of the main body part 14a that has a larger outside diameter, and envelops the flange portion 12b of the core sleeve 12.

The small diameter portion 14c is a substantially cylindrical member on the side of the main body part 14a that has a smaller outside diameter, and envelops the heater 13 disposed on the outer peripheral surface of the main body part 12a of the core sleeve 12.

As shown in Fig. 6, the threaded portion (first threaded portion) 14ca is formed along the lengthwise direction of the cables C on the outer peripheral surface in the middle part of the substantially cylindrical main body part 14a, and a nut member 18 (discussed below) is threaded onto the threaded portion 14ca.

As shown in Fig. 6, the threaded portion 14cb is formed on the outer peripheral surface of the end portion on the internal space S2 side of the main body part 14a, and is fixed in a state in which a threaded portion 16c of the distal end cap 16 (discussed below) has been threaded on.

As shown in Fig. 6, the cut portion 14d is formed so as to split the outer peripheral surface of the substantially cylindrical main body part 14a in two along the axial direction. The core sleeve 12 is then inserted from the distal end of the outer sleeve 14 toward the inner peripheral surface side in a state in which the cut portion 14d is open.

As shown in Fig. 6, the intermediate member 15 is a substantially cylindrical member that is disposed on the side of the outer sleeve 14 where the end (second end) on the internal space S2 side is located, and has substantially the same outside diameter as the outer sleeve 14. The intermediate member 15 has a main body part 15a and slits 15b.

The main body part 15a is a substantially cylindrical member made of PTFE (polytetrafluoroethylene) and is enveloped on the inner peripheral surface side of the distal end cap 16.

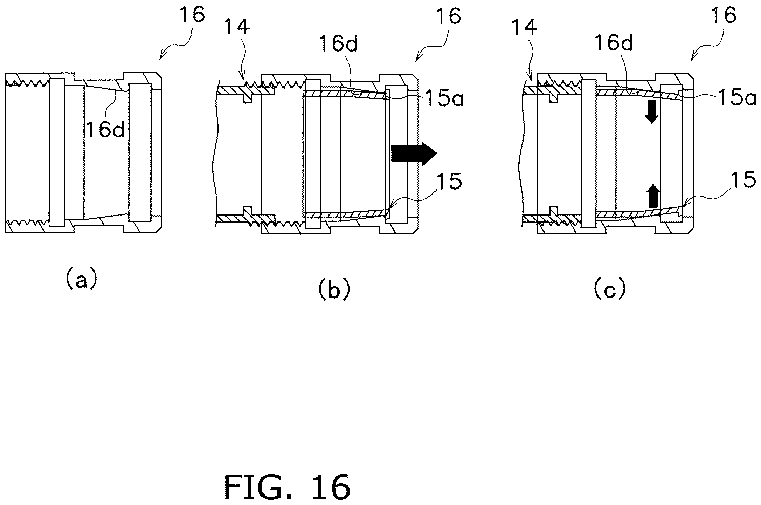

A plurality of the slits 15b are formed around the outer peripheral surface of the main body part 15a and running in the lengthwise direction of the cables C. When the distal end cap 16 is attached to the outer peripheral surface side of the intermediate member 15, an external force is exerted inward in the radial direction by the tapered portion 16d (see Fig. 16A) provided on the inner peripheral surface side of the distal end cap 16, causing the cables C to elastically deform as shown in Figs. 16B and 16C.

This deformation of the intermediate member 15 creates a waterproof structure near the measurement hole 20.

The distal end cap (cap member) 16 is a substantially cylindrical member made of a resin such as PC (polycarbonate) and, as shown in Fig. 6, is attached so as to cover the end on the internal space S2 side of the outer sleeve 14 and the outer peripheral surface of the intermediate member 15. The distal end cap 16 has a main body part 16a, a recess 16b, a threaded portion 16c, and a tapered portion 16d.

The main body part 16a is a substantially cylindrical member.

The recess 16b is a groove into which a sealing member 17 (discussed below) fits, and is provided near the approximate center of the main body part 16a.

The threaded portion 16c meshes with the above-mentioned threaded portion 14cb of the outer sleeve 14. This allows the distal end cap 16 to be fixed to the end on the internal space S2 side of the outer sleeve 14.

The tapered portion 16d is provided on the inner peripheral surface side of the substantially cylindrical main body part 16a, and as shown in Fig. 16A, is formed so that its inside diameter decreases toward the internal space S2 side.

Consequently, when the distal end cap 16 is attached to the distal end of the outer sleeve 14, the tapered portion 16d comes into contact with the outer peripheral surface of the main body part 15a of the intermediate member 15 and presses it toward the inside diameter side, as shown in Fig. 16B. Therefore, when the mounting of the distal end cap 16 to the distal end of the outer sleeve 14 is complete, the portion of the main body part 15a of the intermediate member 15 where the tapered portion 16d comes into contact elastically deforms toward the inside diameter side, as shown in Fig. 16C.

Consequently, a waterproofing mechanism can be formed in the measurement hole 20 by the elastic deformation of the main body part 15a of the intermediate member 15 as the distal end cap 16 is mounted to the distal end of the outer sleeve 14.

As shown in Fig. 6, the sealing member 17 is, for example, a substantially annular member made of silicone rubber, which is mounted to the outer peripheral surface of the distal end cap 16 and has an elastic main body part 17a and a cut portion 17b. The sealing member 17 is inserted into the measurement hole 20 to seal the space on the indoor side from the outdoor side.

The main body part 17a is a substantially annular elastically deformable member, and is attached to the recess 16b of the distal end cap 16 in a state in which the cut portion 17b has been opened.

The cut portion 17b is formed so as to split the outer peripheral surface of the substantially annular main body part 17a in two along the axial direction. Consequently, the main body part 17a can be attached to the recess 16b of the distal end cap 16 by opening the cut portion 17b while elastically deforming the main body part 17a.

The nut member 18 is a fastening member made of polycarbonate (PC) or another such resin, and as shown in Fig. 6, has a substantially annular main body part 18a and a threaded portion (second threaded portion) 18b that is formed on the inner peripheral side of the main body part 18a and meshed with the threaded portion 14ca on the outer sleeve 14. The nut member 18 is fixed at a position according to the length of the measurement hole 20 in a state in which the threaded portion 18b is meshed with the threaded portion 14ca on the outer sleeve 14 side.

Here, the threaded portion 18b of the nut member 18 meshes with the threaded portion 14ca formed near the center of the main body part 14a of the outer sleeve 14, and therefore does not mesh with the threaded portion 14cb formed at the end on the internal space S2 side.

That is, since the threaded portion 14cb has a smaller outside diameter than the threaded portion 14ca, the nut member 18 can be inserted into the threaded portion 14ca side without meshing with the threaded portion 18b of the nut member 18.

### Assembly of Cable Cover 10

The process of assembling the cable cover 10 in this embodiment will now be described with reference to Figs. 7 to 14.

On the external space S1 side of the cables C passed from the internal space S2 of the constant-temperature unit 30 to the external space S1, the grommet 11 is attached so as to hold the outer peripheral surface of the cables C, as shown in Fig. 7.

At this point, the main body part 11a of the grommet 11 is elastically deformed, and the cut portions 11ba formed in the four holding portions 11b are opened so that the cables C can be held in the holding portions 11b.

Of the four holding portions 11b of the grommet 11, a plug 11c is inserted into any holding portion 11b that does not hold a cable C, as shown in Fig. 7.

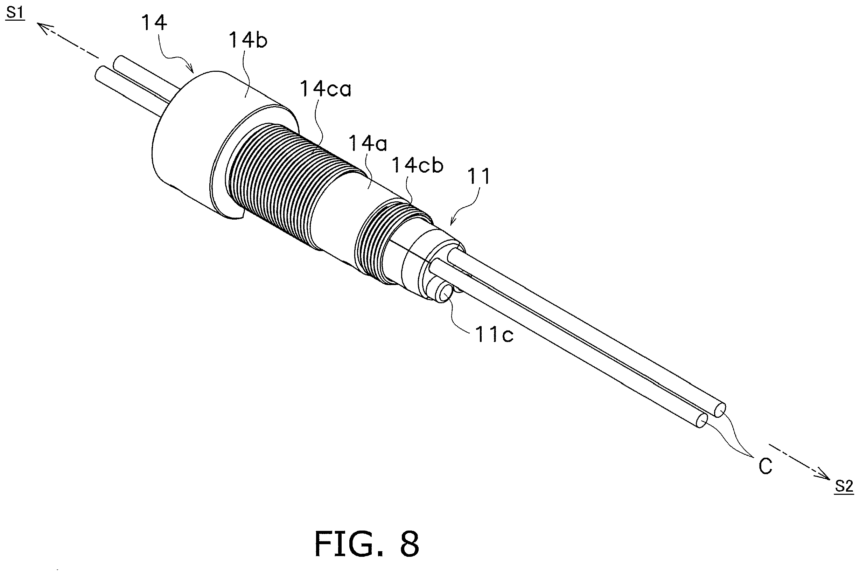

Next, as shown in Fig. 8, sleeve members (core sleeve 12 and outer sleeve 14) are mounted so that the grommet 11 holding the cables C covers the outer periphery.

At this point, the grommet 11 is inserted from the distal end of the core sleeve 12 and is held on the inner peripheral surface side.

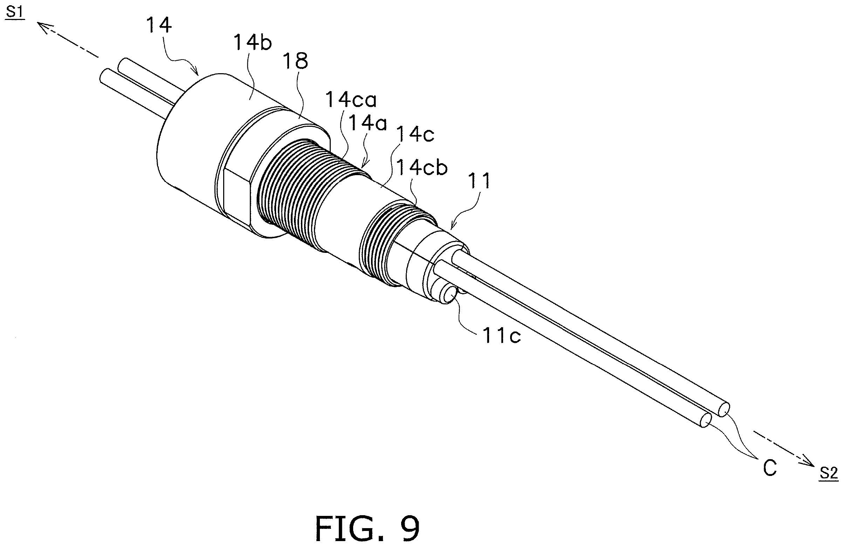

Next, as shown in Fig. 9, the nut member 18 is attached so as to mesh with the threaded portion 14ca provided on the outer periphery of the outer sleeve 14.

At this point, the nut member 18 turns and moves to the end on the external space S1 side in a state of being meshed with the threaded portion 14ca.

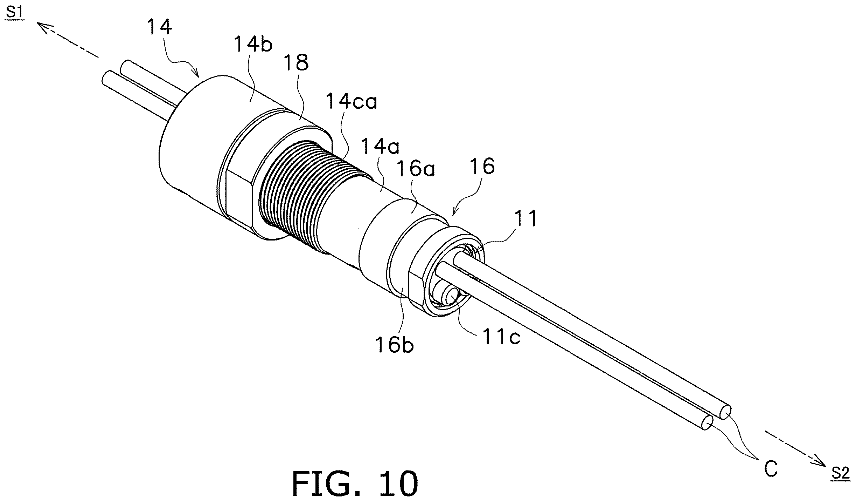

Next, as shown in Fig. 10, the distal end cap 16 is attached to the end of the small diameter portion 14c in the main body part 14a of the outer sleeve 14. The distal end cap 16 is fixed when the threaded portion 16c formed on its inner peripheral surface meshes with the threaded portion 14cb formed on the end of the small diameter portion 14c.

Next, the components shown in Fig. 10 are inserted from the external space S 1 side into the measurement hole 20 fixed in the recess 32a in the rear surface 32 of the housing 31 of the constant-temperature unit 30.

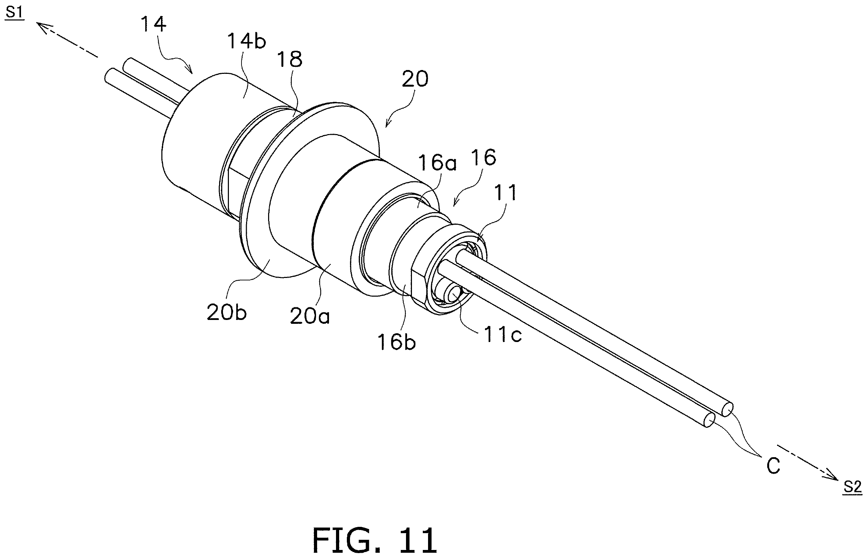

At this point, the components in Fig. 10 are inserted until the nut member 18 attached to the threaded portion 14ca of the large diameter portion 14b of the outer sleeve 14 hits the flange portion 20b of the measurement hole 20, as shown in Fig. 11.

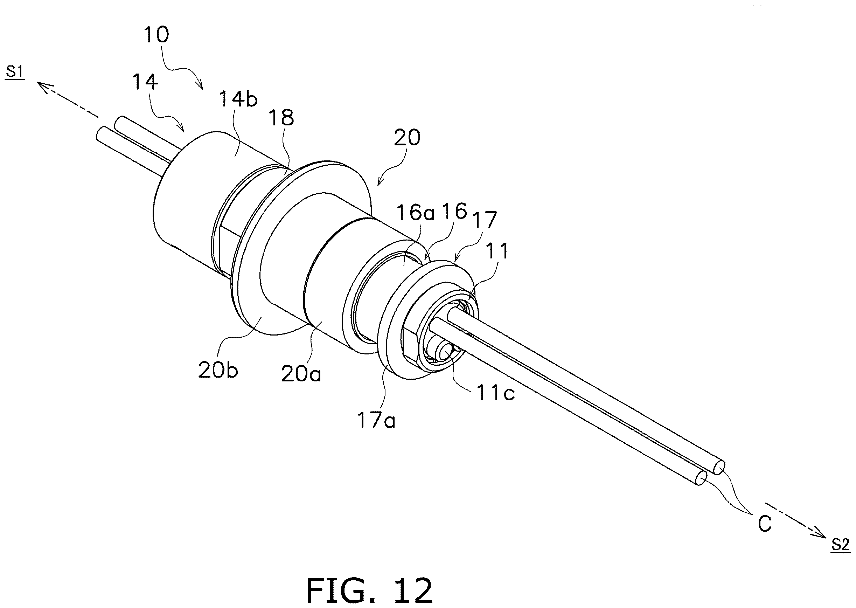

Next, as shown in Fig. 12, the cut portion 17b of the main body part 17a is opened from the internal space S2 side and the sealing member 17 is attached in the recess 16b of the main body part 16a of the distal end cap 16.

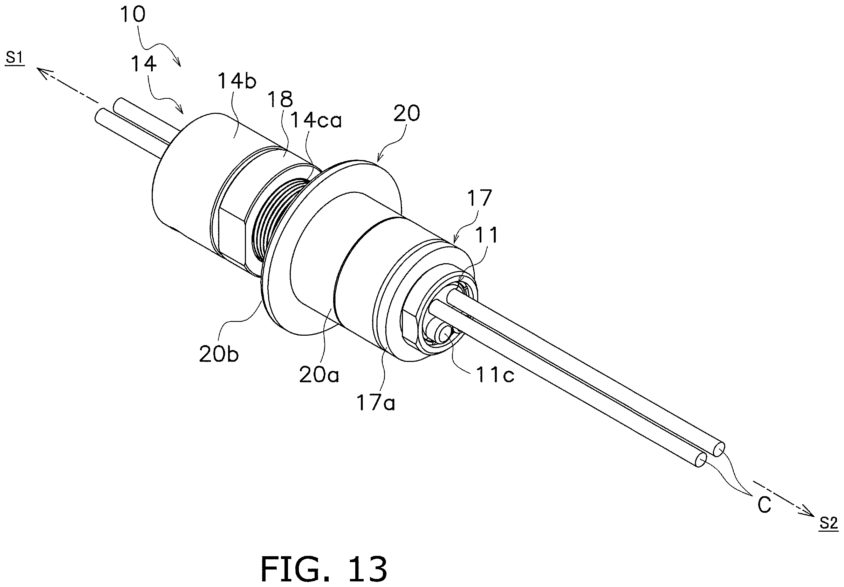

Next, from the state shown in Fig. 12, the outer sleeve 14 is pulled to the external space S1 side as shown in Fig. 13 to change its position relative to the measurement hole 20.

At this point, the end of the main body part 20a on the internal space S2 side of the measurement hole 20 moves to a position where it hits the main body part 17a of the sealing member 17.

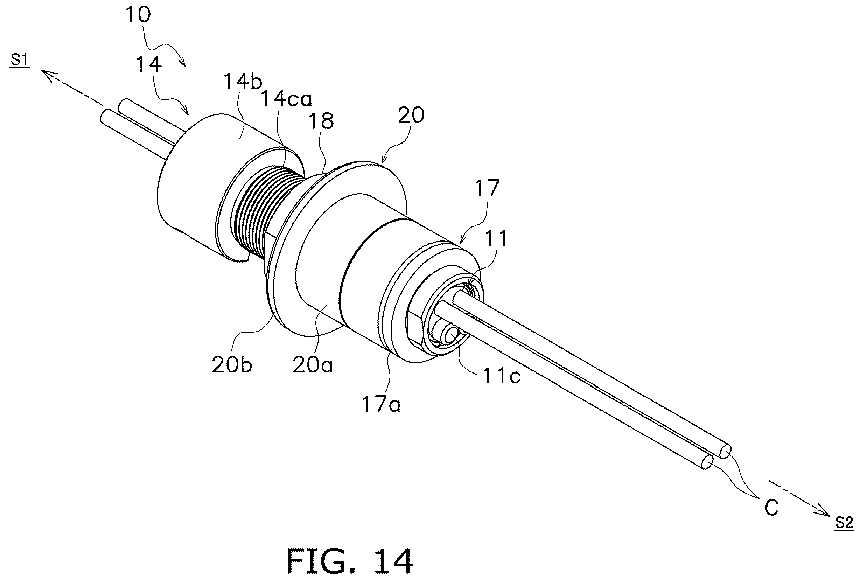

Next, from the state shown in Fig. 13, the nut member 18 is turned to move over the threaded portion 14ca of the outer sleeve 14 to the internal space S2 side as shown in Fig. 14, and the nut member 18 is fixed at a position where it presses against the flange portion 20b of the measurement hole 20.

As a result, the cable cover 10 is fixed to the measurement hole 20 attached to the rear surface 32 of the housing 31 of the constant-temperature unit 30.

Then, in a state in which the cable cover 10 is holding a plurality of cables C, and is fixed with respect to the measurement hole 20 attached to the rear surface 32 of the constant-temperature unit 30, current is sent to the heater 13, which heats the cables C near the measurement hole 20.

This to more effectively prevents condensation from occurring near the measurement hole 20 in the cable cover 10, which fixes the cables C that have been passed from the internal space S2 of the constant-temperature unit 30 to the external space S1 through the measurement hole 20 provided on the rear surface 32 of the constant-temperature unit 30, to the measurement hole 20.

Also, in the constant-temperature unit 30 or other such constant-temperature unit to which the cable cover 10 of this embodiment is attached, the length of the measurement hole 20 may vary from one device to the next.

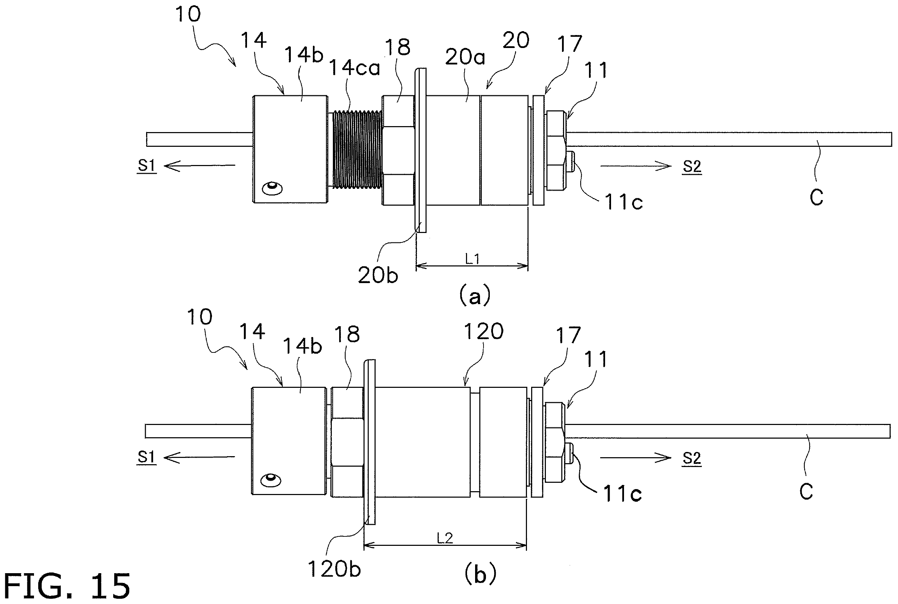

In view of this, as shown in Figs. 15A and 15B, the cable cover 10 of this embodiment can be attached to both of the measurement holes 20 and 120, which have different lengths, by changing the fixing position of the nut member 18 that meshes with the threaded portion 14ca provided on the outer peripheral surface of the outer sleeve 14.

More precisely, if we let L1 be the length of the measurement hole 20 and let L2 be the length of the measurement hole 120, when L1 < L2, as shown in Fig. 15A, the cable cover 10 is fixed when the nut member 18 moves a long distance to the position where it hits the flange portion 20b for a short measurement hole 20.

Similarly, as shown in Fig. 15B, the cable cover 10 is fixed when the nut member 18 moves a short distance to the position where it hits the flange portion 120b for a long measurement hole 120.

Consequently, the cable cover 10 of this embodiment can be attached to either of the measurement holes 20 and 120 attached to the constant-temperature unit 30 or other such constant-temperature unit, even though the measurement holes 20 and 120 have different lengths.

### Embodiment 2

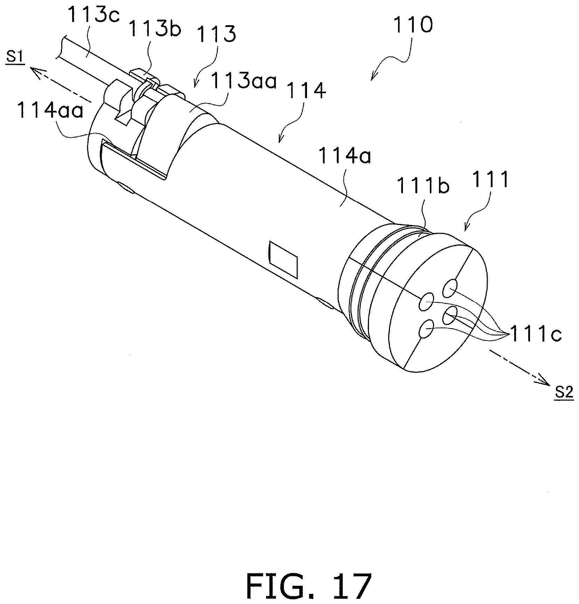

A cable cover 110 according to another embodiment of the present invention will now be described with reference to Figs. 17 to 19. Figs. 17 to 19 show a state in which the cables C are not held in the cable cover 110.

As in Embodiment 1 above, the cable cover 110 of this embodiment fixes cables C (see Fig. 7, etc.) that are passed from the internal space S2 to the external space S1 through a measurement hole 20 attached to the constant-temperature unit 30 or other such constant-temperature unit.

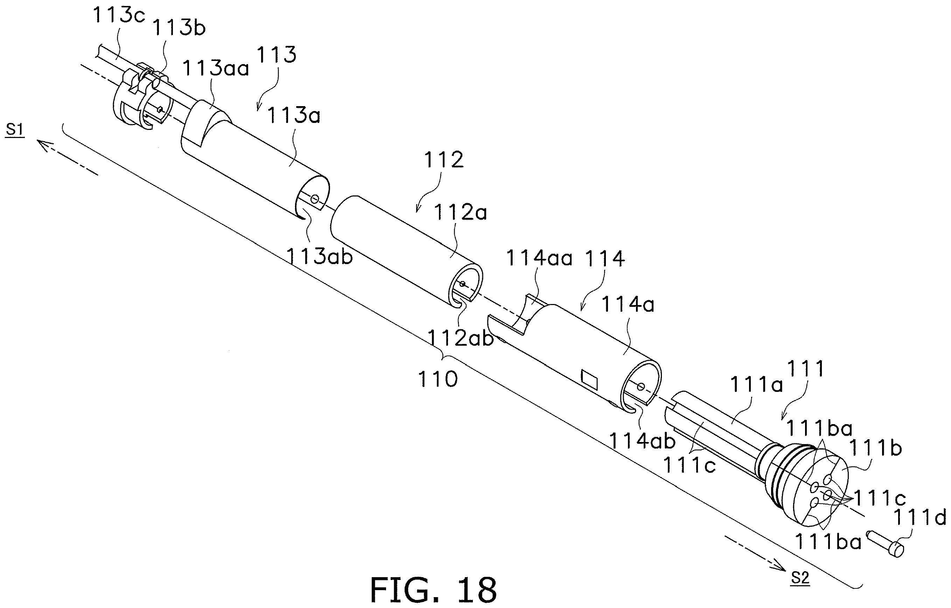

As shown in Figs. 17 and 18, the cable cover 110 has a grommet (holding member) 111, a core sleeve (first sleeve member) 112, a sheet heater (heating unit) 113, and an outer sleeve (second sleeve member) 114.

The grommet (holding member) 111 is, for example, a member made of silicone rubber, which envelops the cables C while being elastically deformed, and holds the outer peripheral portion of the cables C. The grommet 111 has a main body part 111a that constitutes the center portion of the cable cover 110, a cylindrical portion 111b that is linked to the main body part 111a and has a larger outside diameter than the main body part 111a, and a holding portion 111c that holds four cables C (see Fig. 7, etc.).

Also, the grommet 111 has cut portions 111ba that are provided on the outer peripheral surface of the cylindrical portion 111b and that elastically deform when the cables C are held by the holding portion 111c, thereby moving the cables C to the holding portion 111c.

Of the holding portions 111c that hold the four cables C, any holding portion 111c that does not hold a cable C is blocked off with a resin plug (blocking member) 111d. This makes it less likely that the internal space S2 of the constant-temperature unit 30 and the external space S1 will communicate with each other via any holding portions 111c that do not hold cables C.

In this embodiment, a configuration is described in which four cables C are held in the grommet 111, but the number of cables C held, that is, the number of holding portions 111c, is not limited to four.

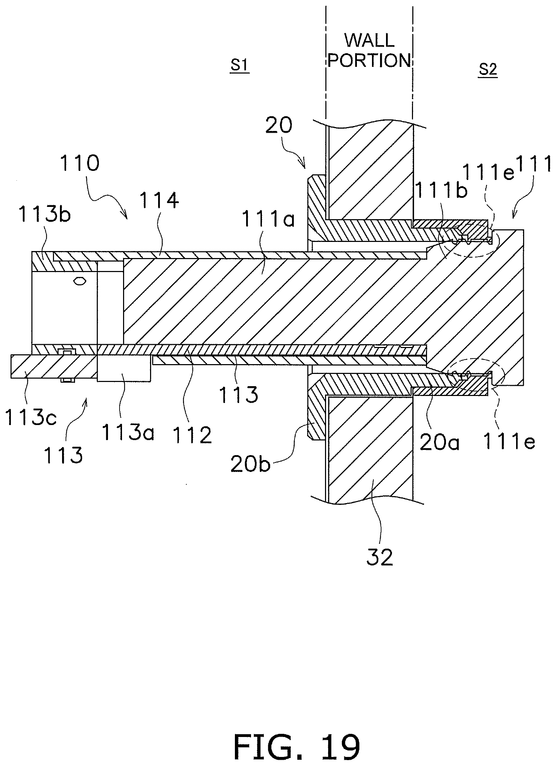

Furthermore, as shown in Fig. 19, the grommet 111 has a press-fit portion 111e that hits the inner peripheral surface of the measurement hole 20 when installed in the measurement hole 20, and is held in place by the elastic deformation of the cylindrical portion 111b.

As shown in Fig. 19, when the cylindrical portion 111b is inserted into the measurement hole 20 from the internal space S2 side, it elastically deforms at the part of the press-fit portion 111e provided on the outer peripheral surface, exerting an elastic force outward in the radial direction.

This allows the outer peripheral surface of the cylindrical portion 111b to be held on the inner peripheral surface of the measurement hole 20.

The core sleeve (first sleeve member) 112 is a member that is made by cutting aluminum, for example, and is disposed on the outer peripheral side of the grommet 111, and as shown in Fig. 18, has a substantially cylindrical main body part 112a and a cut portion 112ab provided along the lengthwise direction of the main body part 112a.

The cut portion 112ab is a gap formed along the lengthwise direction so as to split the substantially cylindrical main body part 112a in the peripheral direction.

The sheet heater (heating unit) 113 is disposed on the outer peripheral surface of the main body part 112a of the core sleeve 112 as shown in Fig. 19 in order to heat a part of the cables C near the measurement hole 20, and applies heat to the cables C held in the grommet 111 enveloped by the core sleeve 112.

This heats the outer peripheral surface of the cables C near the measurement hole 20. Consequently, even if the temperature of the external space S1 is 25 degrees or lower, for example, it is possible to suppress the occurrence of condensation on the outer peripheral surface of the cables C near the measurement hole 20 and near the measurement hole 20 in the internal space S2 by heating the surface of the cables C to a temperature equal to or higher than the temperature of the internal space S2.

As shown in Fig. 18, the sheet heater 113 has a main body part 113a, a retainer 113b, and a heater wire 113c.

The main body part 113a is a sheet-like member and has a connection portion 113aa that is connected to the heater wire 113c, and a cut portion 113ab that is formed along the lengthwise direction so as to split the substantially cylindrical main body part 113a in the peripheral direction.

The retainer 113b is a member that latches the heater wire 113c so that this wire will not come loose, and is mounted on the external space S1 side of the main body part 113a.

The heater wire 113c is connected to the connection portion 113aa of the main body 113a, is provided in order to supply power to the sheet heater 113, and is passed out to the external space S1. If the temperature of the outer surface of the cables C to be heated exceeds a specific temperature setting, for example, the supply of power from the heater wire 113c to the sheet heater 113 is halted, and if the temperature falls below the temperature setting, the supply of power to the sheet heater 113 is started.

The outer sleeve (second sleeve member) 114 is formed, for example, by cutting a resin, and is a member that is disposed on the outer peripheral surface side of the core sleeve 112 and the sheet heater 113. As shown in Fig. 18, the outer sleeve 114 has a substantially cylindrical main body part 114a, an arc portion 114aa that is provided at the end of the main body part 114a on the external space S1 side, and a cut portion 114ab that is provided along the lengthwise direction of the main body part 114a.

The arc portion 114aa is provided at the end of the main body part 114a on the external space S1 side, is formed so that the end of the main body part 114a is split in the peripheral direction, and is a substantially arc-shaped portion when viewed from the external space S1 side. The connecting portion 113aa is disposed so as to be in contact with the portion that is split in the peripheral direction where the arc portion 114aa is provided, as shown in Figs. 17 and 19.

The cut portion 114ab is formed along the lengthwise direction so as to split the substantially cylindrical main body part 114a in the peripheral direction. When the grommet 111 to which the cables C are attached is put in place, the grommet 111 is inserted from the distal end side of the outer sleeve 114 and is held on the inner peripheral surface side of the main body part 114a.

With the cable cover 110 in this embodiment, as discussed above, the grommet 111 has the press-fit portion 111e that hits the inner surface of the measurement hole 20 when installed in the measurement hole 20 and is held in place by the elastic deformation of the cylindrical portion 111b (see Fig. 19).

This makes use of the elasticity of the grommet 111 to press the outer surface of the grommet 111 into contact with the inner peripheral surface of the measurement hole 20, so that the cable cover 110 can be easily fixed to the measurement hole 20 without having to use any screws or other such fixing members.

Also, as shown in Fig. 19, the sheet heater 113 is disposed around the outer peripheral surface of the main body part 112a of the core sleeve 112, and applies heat to the cables C held in the grommet 111 contained enveloped by the core sleeve 112.

This allows a portion of the cables C near the measurement hole 20 to be heated, which effectively prevents condensation from forming on the surface of the cables C.

### Embodiment 3

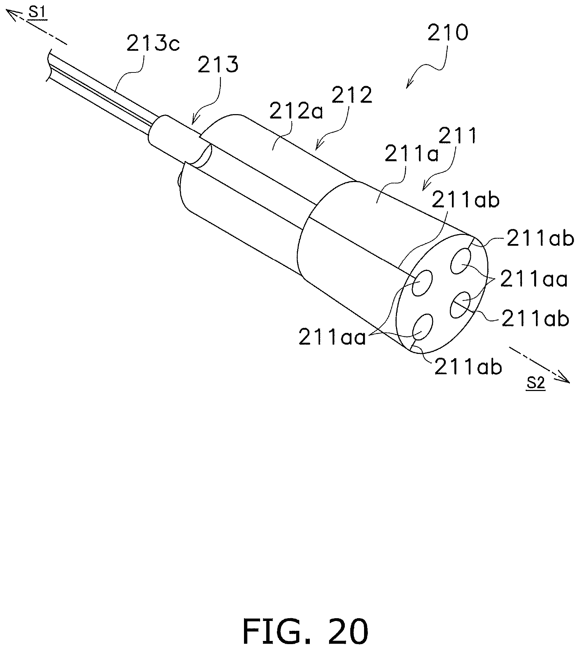

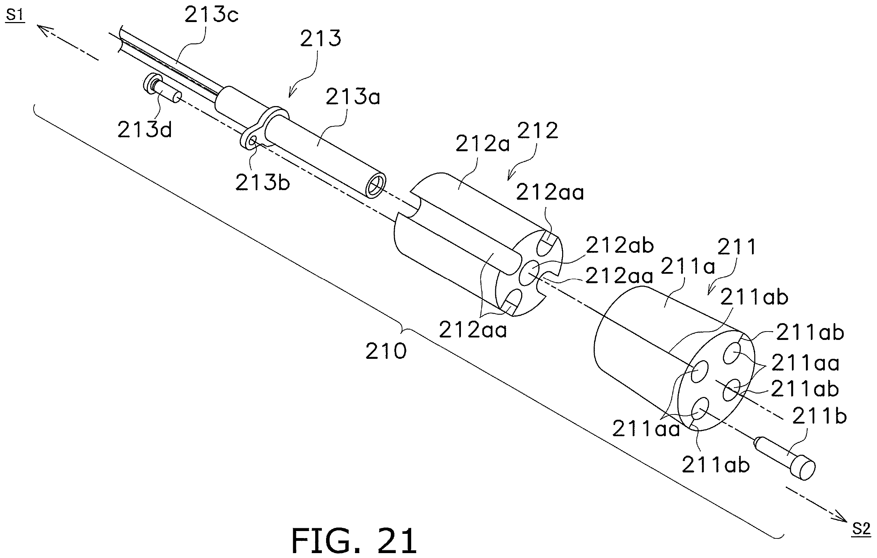

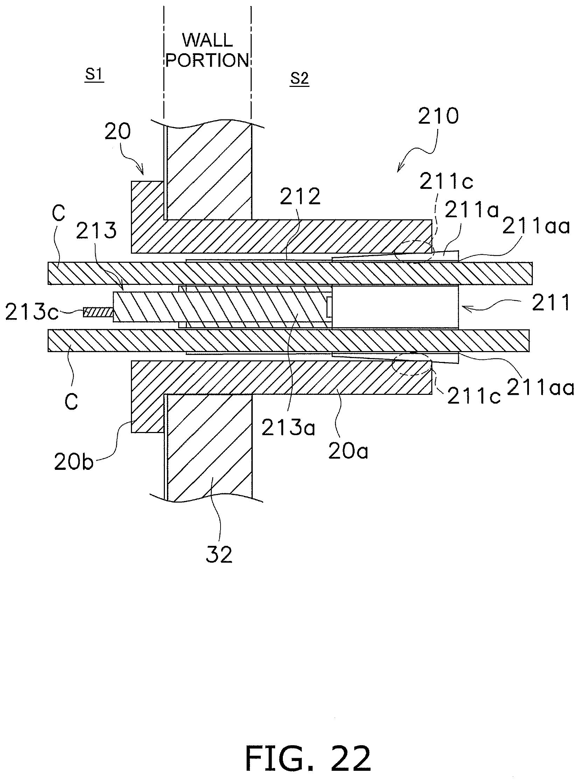

A cable cover 210 according to yet another embodiment of the present invention will now be described with reference to Figs. 20 to 22. Figs. 20 and 21 show the cable cover 210 in a state of not holding any cables C, and Fig. 22 shows the cable cover 210 in a state of holding the cables C.

The cable cover 210 of this embodiment, just as with the cable covers 10 and 110 of the above Embodiments 1 and 2, fixes cables C (see Fig. 22, etc.) that are passed from the internal space S2 to the external space S1 through a measurement hole 20 attached to an incubator 30 or other such constant-temperature unit.

As shown in Figs. 20 and 21, the cable cover 210 has a silicone plug (second holding member) 211, an aluminum block (first holding member) 212, and a cartridge heater (heating unit) 213.

As shown in Fig. 20, the silicone plug (second holding member) 211 is disposed the closest to the internal space S2 of all the members constituting the cable cover 210. The silicone plug 211 is disposed so as to be in contact with the aluminum block (first holding member) 212 in the direction in which the cables C extend, and has a main body part 211a, holding portions 211aa, and cut portions 211ab.

The main body part 211a is a substantially cylindrical elastic member made of silicone rubber, for example.

The holding portions 211aa are four through-holes provided along the lengthwise direction of the main body part 211a, and hold the cables C. This allows the cable cover 210 to hold four cables C in the four holding portions 211aa.

The cut portions 211ab are provided on the outer peripheral surface of the main body part 211a, and elastically deform to open when the cables C are to be held in the holding portions 211aa, thereby moving the cables C to the holding portions 211aa.

Among the holding portions 211aa that hold the four cables C, any holding portion 211aa that does not hold a cable C is blocked off with a resin plug (blocking member) 211b. This prevents the internal space S2 of the constant-temperature unit 30 and the external space S1 from communicating with each other through holding portions 211aa that do not hold cables C.

Also, the silicone plug 211 has a press-fit portion 211c that hits the inner peripheral surface of the measurement hole 20 when installed in the measurement hole 20 and is held in place by elastic deformation.

The silicone plug 211 hits the inner peripheral surface of the measurement hole 20 when installed in the measurement hole 20, and is held in place by the elastic deformation of the main body part 211a (see Fig. 22).

This makes use of the elasticity of the silicone plug 211 to press the outer surface of the silicone plug 211 into contact with the inner surface of the measurement hole 20, so that the cable cover 210 can be easily fixed to the measurement hole 20.

The aluminum block (first holding member) 212 is formed by cutting a block of aluminum, and has a substantially cylindrical main body part 212a, a plurality of holding portions 212aa provided to the main body part 212a, and a substantially cylindrical insertion hole 212ab provided in the center of the main body part 212a.

As shown in Fig. 21, the insertion hole 212ab is a through-hole formed along the center axis of the substantially cylindrical main body part 212a, and has an inside diameter that is slightly larger than the outside diameter of the main body part 213a of the cartridge heater 213. The cartridge heater 213 is inserted into the insertion hole 212ab.

The holding portions 212aa are provided on the outer peripheral side of the insertion hole, and each holds a cable C. In this embodiment, a configuration is described in which four cables C are held in the aluminum block 212, but the number of cables C held, that is, the number of holding portions 212aa, is not limited to four.

The aluminum block 212 has a plurality of holding portions 212aa provided on the outer peripheral surface of the main body part 212a.

As shown in Fig. 21, the holding portions 212aa are provided along the lengthwise direction at approximately equal angular intervals on the outer peripheral surface of the substantially cylindrical main body part 212a, and are recesses into which the cables C are fitted from the outer peripheral side.

The aluminum block 212 is formed from a metal material having high thermal conductivity, such as aluminum. Consequently, the heat coming off the cartridge heater 213 can be efficiently transferred to the cables C.

As shown in Fig. 21, the cartridge heater (heating unit) 213 is a rod-shaped heating element, and as shown in Fig. 22, is inserted into the insertion hole 212ab formed along the center axis of the aluminum block 212. The cartridge heater 213 has a main body part 213a, a flange portion 213b, a heater wire 213c, and a screw 213d.

The main body part 213a is a substantially cylindrical rod-shaped member, and is connected to the heater wire 213c.

The flange portion 213b protrudes outward in the radial direction from the outer peripheral surface of the substantially cylindrical main body part 213a, and is fixed to the end surface of the aluminum block 212 by means of the screw 213d.

The flange portion 213b may be fixed to the aluminum block 212 by some other means besides a screw, such as an adhesive agent.

The heater wire 213c is connected to the main body part 213a, is provided in order to supply power to the cartridge heater 213, and is passed through to the external space S1 side. If the temperature of the outer surface of the cables C to be heated exceeds a specific temperature setting, for example, the power supply from the heater wire 213c to the cartridge heater 213 is halted, and if the temperature falls below the temperature setting, the power supply to the cartridge heater 213 is started.

As described above, the cable cover 210 of this embodiment has a press-fit portion 211c in which the silicone plug 211 comes into contact with the inner surface of the measurement hole 20 when installed in the measurement hole 20, and is held in place by the elastic deformation of the main body part 211a (see Fig. 22).

This makes use of the elasticity of the silicone plug 211 to press the outer surface of the silicone plug 211 into contact with the inner peripheral surface of the measurement hole 20, so that the cable cover 210 can be easily fixed to the measurement hole 20 without having to use any screws or other such fixing members.

As shown in Fig. 22, etc., the cartridge heater 213 is disposed near the center axis of the main body part 212a of the aluminum block 212, and applies heat to the cables C held on the outer peripheral surface side of the aluminum block 212.

This allows a portion of the cables C near the measurement hole 20 to be heated, which effectively prevents condensation from forming on the surface of the cables C.

### Other Embodiments

Embodiments of the present invention were described above, but the present invention is not limited to or by the above embodiments, and various modifications are possible without departing from the gist of the invention.
(A) In the above embodiments, an example was given in which the sleeve member employed a two-layer structure including the core sleeve 12 and the outer sleeve 14. However, the present invention is not limited to this.
   For example, the sleeve member may be made up of just a single member.
(B) In the above embodiments, an example was given in which a substantially cylindrical member (measurement hole 20) was fixed to the rear surface (wall surface) 32 on the constant-temperature unit 30 side to which the cable cover 10 was attached. However, the present invention is not limited to this.
   For example, components on the constant-temperature unit side where the cable cover is attached may be directly attached to the wall surface.
(C) In the above embodiments, an example was given in which the sheet-shaped heater 13 was wrapped around the outer peripheral surface of the core sleeve 12 in order to prevent condensation from occurring near the measurement hole 20. However, the present invention is not limited to this.
   For example, the heating unit used to prevent condensation may be in some form other than a sheet.
(D) In the above embodiments, an example was given in which the cable cover 10 held four cables C. However, the present invention is not limited to this.
   For example, the cable cover may be configured to hold one to three cables, or configured to hold five or more cables.
(E) In the above embodiments, an example was given in which the constant-temperature unit 30 that performed cell culture was used as the constant-temperature unit to which the cable cover 10 of the embodiments was attached. However, the present invention is not limited to this.
   For example, the constant-temperature unit to which the cable cover is attached is not limited to being an incubator, and may instead be any of various kinds of constant-temperature unit used in environments of high temperature and/or high humidity.
(F) In Embodiment 3 above, an example was given in which the silicone plug (second holding member) 211 and the aluminum block (first holding member) 212 were provided as separate members. However, the present invention is not limited to this.

For example, the first holding member and the second holding member may be configured as an integral member.

Here again, the occurrence of condensation on the surface of the cables can be effectively suppressed by heating the cables with a heater or other such heating unit while the cables are held in a member that integrates the first and second holding members.

In this case, the cable cover can be easily fixed to the measurement hole by press-fitting, just as in Embodiments 2 and 3 above, by providing an elastic member on the inner peripheral side of the measurement hole 20 or on the outer peripheral side a member formed by integrating a first holding member and a second holding member, for example.

### INDUSTRIAL APPLICABILITY

The cable cover of the present invention is a cable cover that fixes a cable that is passed from the inside to the outside through an opening provided in the wall of a constant-temperature unit, and exhibits the effect that the occurrence of condensation near the opening can be more effectively prevented, and as such can be widely applied to a variety of devices in which cables are passed from an indoor side to an outdoor side.

### REFERENCE SIGNS LIST

- 10: cable cover
- 11: grommet (holding member)
- 11a: main body part
- 11b: holding portion
- 11ba: cut portion
- 11c: plug (blocking member)
- 12: core sleeve (first sleeve member, sleeve member)
- 12a: main body part
- 12b: flange portion
- 12c: cut portion
- 13: heater (heating unit)
- 13a: heater wire
- 14: outer sleeve (second sleeve member, sleeve member)
- 14a: main body part
- 14b: large diameter portion
- 14c: small diameter portion
- 14ca: threaded portion (first threaded portion)
- 14cb: threaded portion
- 14d: cut portion
- 15: intermediate member
- 15a: main body part
- 15b: slit
- 16: distal end cap (cap member)
- 16a: main body part
- 16b: recess
- 16c: threaded portion
- 16d: tapered portion
- 17: sealing member
- 17a: main body part
- 17b: cut portion
- 18: nut member (fixing member)
- 18a: main body part
- 18b: threaded portion (second threaded portion)
- 20: measurement hole (installation hole)
- 20a: main body part
- 20b: flange portion
- 30: constant-temperature unit (constant-temperature unit)
- 31: housing
- 32: rear surface (wall surface)
- 32a: recess
- 110: cable cover
- 111: grommet (holding member)
- 111a: main body part
- 111b: cylindrical portion
- 111ba: cut portion
- 111c: holding portion
- 111d: plug (blocking member)
- 111e: press-fit portion
- 112: core sleeve (first sleeve member)
- 112a: main body part
- 112ab: cut portion
- 113: sheet heater (heating unit)
- 113a: main body part
- 113aa: connection portion
- 113ab: cut portion
- 113b: retainer
- 113c: heater wire
- 114: outer sleeve (second sleeve member)
- 114a: main body part
- 114aa: arc portion
- 114ab: cut portion
- 120: measurement hole (installation hole)
- 120b: flange portion
- 210: cable cover
- 211: silicone plug (second holding member)
- 211a: main body part
- 211aa: holding portion
- 211ab: cut portion
- 211b: plug (blocking member)
- 211c: press-fit portion
- 212: aluminum block (first holding member)
- 212a: main body part
- 212aa: holding portion
- 212ab: insertion hole
- 213: cartridge heater (heating unit)
- 213a: main body part
- 213b: flange portion
- 213c: heater wire
- 213d: screw
- C: cable
- S1: external space
- S2: internal space

## Claims

1. A cable cover that fixes, in an installation hole provided in a wall surface of a constant-temperature unit, a cable coming out of an internal space, through the installation hole, and into an external space, the cable cover comprising:
a holding member configured to hold the cable;
a substantially cylindrical first sleeve member that is disposed around an outer periphery of the holding member; and
a heating unit that is disposed on the first sleeve member and is configured to apply heat to the cable held via the first sleeve member by the holding member.

2. A cable cover that fixes, in an installation hole provided in a wall surface of a constant-temperature unit, a cable coming out of the internal space, through the installation hole, and into the external space, the cable cover comprising:
a first holding member configured to hold the cable;
a second holding member that is disposed so as to come into contact with the first holding member each other in an extension direction of the cable; and
a heating unit that is disposed on the first holding member and is configured to apply heat to the cable held by the first holding member via the first holding member.

3. A cable cover that fixes, in an installation hole provided in a wall surface of a constant-temperature unit, a cable coming out of the internal space, through the installation hole, and into the external space, the cable cover comprising:
a holding member configured to hold the cable;
a substantially cylindrical sleeve member that is disposed around an outer periphery of the holding member and has a first threaded portion formed on an outer peripheral surface of the sleeve member along a lengthwise direction of the cable; and
a fixing member that has a substantially annular main body part and a second threaded portion formed on an inner peripheral side of the main body part and meshing with the first threaded portion, and that is fixed at a position corresponding to a length of the installation hole in a state in which the second threaded portion is meshed with the first threaded portion.

4. The cable cover according to claim 1,
further comprising a substantially cylindrical second sleeve member that is attached so as to cover the outer peripheries of the first sleeve member and the heating unit.

5. The cable cover according to claim 4,
wherein the second sleeve member has a first threaded portion that is formed on the substantially cylindrical outer peripheral surface along a lengthwise direction of the cable, and
the cable cover further comprises a fixing member that has a substantially annular main body part and a second threaded portion formed on an inner peripheral side of the main body part and meshing with the first threaded portion, and that is fixed at a position corresponding to a length of the installation hole in a state in which the second threaded portion is meshed with the first threaded portion.

6. The cable cover according to claim 4,
wherein the second sleeve member has a first end on an outdoor side of the constant-temperature unit, and a second end on an indoor side,
the cable cover further comprising an intermediate member that is disposed on a side of the second end of the second sleeve member and has substantially the same outside diameter as the second sleeve member; and a cap member that is attached so as to cover a second end of the second sleeve member and an outer peripheral surface of the intermediate member.

7. The cable cover according to claim 6,
wherein mounting of the cap member causes the intermediate member to be deformed, forming a waterproofing mechanism in the installation hole.

8. The cable cover according to claim 6 or 7,
further comprising a sealing member that is mounted to an outer peripheral surface of the cap member, is elastic, and is inserted into the installation hole to seal off the internal space.

9. The cable cover according to claim 1 or 3,
wherein the holding member has a plurality of holding portions that hold a plurality of the cables.

10. The cable cover according to claim 9,
further comprising a blocking member configured to block off an end of a holding portion that is not holding the cable, among the plurality of holding portions of the holding member.

11. The cable cover according to claim 1 or 3,
wherein the holding member is elastic.

12. The cable cover according to claim 11,
wherein the holding member has a press-fit portion configured to make contact with an inner peripheral surface of the installation hole when installed in the installation hole, and is held by elastic deformation.

13. The cable cover according to claim 11,
wherein the holding member has a holding portion configured to hold the cable, and a cut portion that is provided in an outer peripheral surface thereof and that elastically deforms when the cable is held by the holding portion, thereby moving the cable to the holding portion.

14. The cable cover according to claim 2,
wherein the first holding member has a substantially cylindrical insertion hole provided in a center thereof, and
the heating unit is inserted into the insertion hole.

15. The cable cover according to claim 14,
wherein the first holding member has a holding portion provided on an outer peripheral side of the insertion hole, and
the cable is held in the holding portion.

16. The cable cover according to claim 15,
wherein the first holding member has a plurality of the holding portions, and
the plurality of holding portions are recesses into which the cables are fitted from an outer peripheral side of the first holding member.

17. The cable cover according to claim 2.
wherein the second holding member is formed by a substantially cylindrical elastic member.

18. The cable cover of claim 17,
wherein the second holding member has a holding portion configured to hold the cable, and a cut portion that is provided on an outer peripheral surface of the holding portion and elastically deforms when the cable is held by the holding portion, thereby moving the cable to the holding portion.

19. The cable cover of claim 17,
wherein the second holding member has a press-fit portion configured to make contact with an inner peripheral surface of the installation hole when installed in the installation hole, and is held by elastic deformation.

20. The cable cover according to claim 2,
wherein the first holding member is formed from a metal material having high thermal conductivity.

21. A constant-temperature unit, comprising:
the cable cover according to any of claims 1 to 3; and
a housing that includes a wall surface in which the installation hole is provided.
